Europäisches Patentamt

European Patent Office

Office européen des brevets

(11) Publication number: **0 061 332**
**B1**

(12) **EUROPEAN PATENT SPECIFICATION**

(45) Date of publication of patent specification: **21.11.85**

(21) Application number: **82301453.5**

(22) Date of filing: **22.03.82**

(51) Int. Cl.⁴: **A 61 B 8/12, A 61 B 1/00**

(54) **Combined endoscope and ultrasonic diagnostic device.**

(30) Priority: **22.03.81 JP 40813/81**
**22.03.82 JP 40814/81**
**22.03.81 JP 40815/81**
**22.03.81 JP 40816/81**
**22.03.81 JP 39286/81 U**

(43) Date of publication of application:
**29.09.82 Bulletin 82/39**

(45) Publication of the grant of the patent:
**21.11.85 Bulletin 85/47**

(84) Designated Contracting States:
**AT BE CH DE FR GB IT LI NL SE**

(56) References cited:
**DE-A-2 950 203**
**FR-A-2 365 324**
**FR-A-2 467 583**
**GB-A-1 078 036**
**US-A-2 079 233**
**US-A-2 867 209**

(73) Proprietor: **OLYMPUS OPTICAL CO., LTD.**
**43-2, 2-chome, Hatagaya Shibuya-ku**
**Tokyo 151 (JP)**

(72) Inventor: **Matsuo, Kazumasa**
**No. 1-7-304, Ochiai 3-chome**
**Tama-shi Tokyo (JP)**
Inventor: **Nakada, Akio**
**No. 13-33, Hiyoshi-cho**
**Hachioji-shi Tokyo (JP)**

(74) Representative: **Kennedy, Victor Kenneth et al**
**G.F. REDFERN & CO. Marlborough Lodge 14**
**Farncombe Road**
**Worthing West Sussex BN11 2BT (GB)**

Courier Press, Leamington Spa, England.

## Description

This invention relates to combined endoscope and ultrasonic diagnostic devices for the examination of internal body cavities, wherein an endoscope is incorporated in the housing of an ultrasonic probe so that the probe tip can be placed in contact with an internal organ for diagnostic purposes, the probe tip having a window for transmitting and receiving ultrasonic waves, and being shaped to permit sighting via the endoscope whilst held in the required diagnostic position.

As a means of obtaining information on an object ultrasonic wave probes have recently come to be extensively used in the medical field. For example, when ultrasonic wave pulse are projected into a body from its surface, the projected waves will be propagated on to be reflected by any discontinuous boundary surface forming an acoustic impedance represented by the product of the density of the medium and the velocity of the sound, and therefore the reflected pulses can be received and information for use in diagnosis.

As compared with an X-ray device, such ultrasonic wave diagnostic devices have the advantages that information on a soft biotic structure can be easily obtained without requiring aids such as x-ray opaque substances or contrast media, that a biotic structure is not destroyed by high energy rays, and that the diagnostic device is easier to handle and is less dangerous. Recent developments in ultrasonic techniques have improved the quality and quantity of the obtained information to such a degree that the ultrasonic probe is becoming popular for use as a clinical diagnostic device.

In comparison with the above mentioned diagnosis by the use of ultrasonic wave pulses transmitted and received from the surface of a body, the method of diagnosis in a body cavity by the use of ultrasonic wave pulses transmitted and received from a position near an internal biotic organ within that body cavity has advantages, in that it is possible to obtain readily analysable high frequency information of high precision, and the diagnosis is not influenced by any hypodermic fat layer or the like that may be interposed, and therefore such probes will be used more in the future. Such body cavity interior ultrasonic wave diagnostic devices, constructed to be used when inserted into a body cavity, generally incorporate an endoscope as a convenient optical observation means.

Known combinations of such diagnostic devices incorporating both an ultrasonic probe and an endoscope include the following disclosures:—

"Probe for Inspecting a Body Cavity Interior" mentioned in the Gazette of Japanese Patent Laid Open No. 94230/1980;

"Body Cavity Interior Inspecting Probe" mentioned in the Gazette of Japanese Patent Laid Open No. 94231/1980;

"Ultrasonic Wave Probe" mentioned in the Gazette of Japanese Patent Laid Open No. 96130/1980; and

"Endoscope" as described in the German Offenlegungschrift No. 29 50 203 published on the 19th June 1980.

However, each of the above-mentioned prior art devices has the disadvantage of a construction such that, when operating, the probe tip can not be viewed via the endoscope to show the accurate position at which the probe tip is to contact an internal organ, or the operating position of the probe tip when in contact with the internal organ.

One object of the present invention is to provide a diagnostic device that combines an endoscope and an ultrasonic probe in such a manner that the field of vision of the endoscope is unobstructed by the insertion sheath, ultrasonic probe tip or its curved part, so that the probe can be accurately and safely inserted and guided into a body cavity while the body cavity interior is being observed through the endoscope, both the probe tip and an internal organ can be simultaneously viewed, and a window provided on the tip for transmitting and receiving ultrasonic waves can be accurately placed in contact with the internal organ in a predetermined position.

One preferred embodiment of the invention takes the form of a combined endoscope and ultrasonic diagnostic device wherein the endoscope is incorporated in a housing carrying an ultrasonic probe, which housing comprises an elongate insertion sheath connected by a bendable curving part to a distal probe tip which has a first, peripheral window for transmitting and receiving ultrasonic waves when in contact with an internal organ within a body cavity, the insertion sheath, probe tip and curving part all being of substantially equal diameter, characterised in that a second, peripheral window is incised in the outer periphery of the insertion sheath proximally of the above-mentioned curving part in a position to give a view of the probe tip and first window, when said curving part is bent, and that the endoscope is housed within the insertion sheath to extend from said second window to the proximal end of the sheath, whereby bending of the curving part may be effected to make the first window visible through the second window.

The invention will now be described with reference to the drawings, in which:

Figure 1 is a side view of one known combined endoscope and ultrasonic diagnostic device;

Figure 2 is an explanatory view of the diagnostic device of Figure 1 in use;

Figure 3 is a simplified perspective view of one exemplary embodiment of the present invention;

Figure 4 is a side view of the endoscope portion pulled out of a probe housing that forms an insertion sheath;

Figure 5 is a view of the sheath probe tip seen in the direction indicated by an arrow A in Figure 4;

Figure 6 is a cross-sectional view of the probe tip and curved part;

Figure 7 is a sectional view showing details of an incised window in the insertion sheath and structure of the curved part and a connecting member;

Figure 8 is a cross-section on the line B—B in Figure 7;

Figure 9 is a cross-section of the insertion sheath;

Figure 10 is a fragmentary section showing details of a hand grip and associated housing parts;

Figure 11 is an explanatory view showing the device in use; and

Figure 12 is an explanatory view showing the field of view of the endoscope in Figure 11.

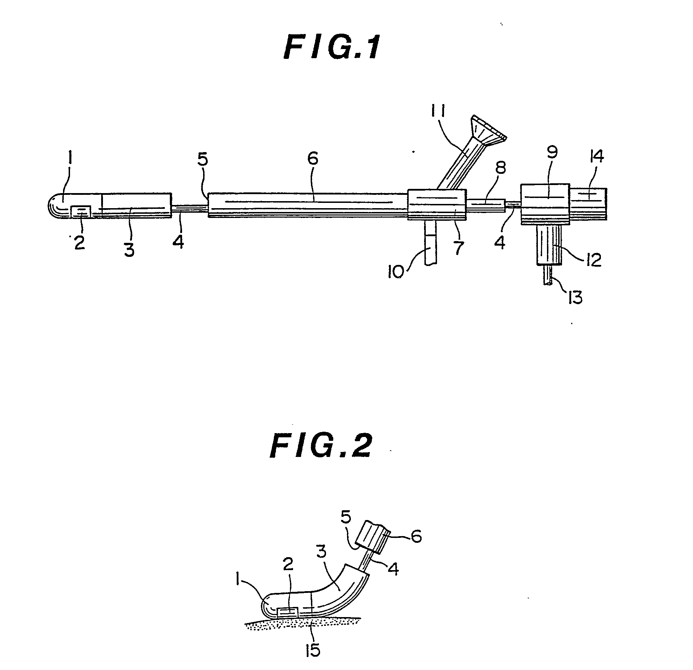

Prior to explaining an embodiment of the present invention, we will describe a known conventional example of a diagnostic device combining an endoscope for observing a body cavity interior and an ultrasonic probe for obtaining a signal image, shown in Figures 1 and 2. In these drawings, an ultrasonic probe tip 1 has a window 2 for transmitting and receiving ultrasonic waves formed in a side of the tip, and a fine diameter shaft 4 is connected to the rear of the tip through a curving part 3. Leads for transmitting and receiving ultrasonic signals, and cables (not illustrated) for driving a mirror within the above mentioned probe tip 1 are contained in this shaft. This shaft 4 is passed through a channel in an insertion sheath 6 which also houses an endoscope 5 and is connected at its rear end to a driving unit 9 through an endoscope hand grip 7 and channel inserting port 8. A light guide cable 10 and an eyepiece 11 inclined to the axial direction of the insertion sheath 6 are connected to the above-mentioned endoscope hand grip 7. A handle member 12 is connected below the driving unit 9. A signal cable 13 is inserted through the handle member 12. A driving motor 14 is connected to the rear of the driving unit 9.

In the above mentioned diagnostic device, in order to obtain an ultrasonic wave image, the insertion sheath 6 and probe tip 1 are inserted into a body cavity, the probe tip 1 is pressed down in contact with a predetermined internal organ 15 visible within the field of vision of the endoscope 5 in the insertion sheath 6, and the curving part 3 can then be curved as shown in Figure 2 so as to bring the window 2 of the probe tip 1 into contact with the internal organ 15.

However, in the above mentioned conventional device, the probe tip 1 and curving part 3 project so far forward from the tip of the endoscope 5 that, when inserted into a body cavity and the probe tip 1 brought into close contact with the internal organ 15, the observed field of vision of the endoscope 5 will be intercepted, the inserted part will have to be pressed down to bend the curving part 3 and bring the tip into close contact with the organ, and the complex operation requires a skilled technique, which is disadvantageous.

In other constructions described in the prior art listed above, the viewing window is closely adjacent the transmitting window, at the tip end of the curving part, so that there is no visibility when the tip is in contact with the internal organ.

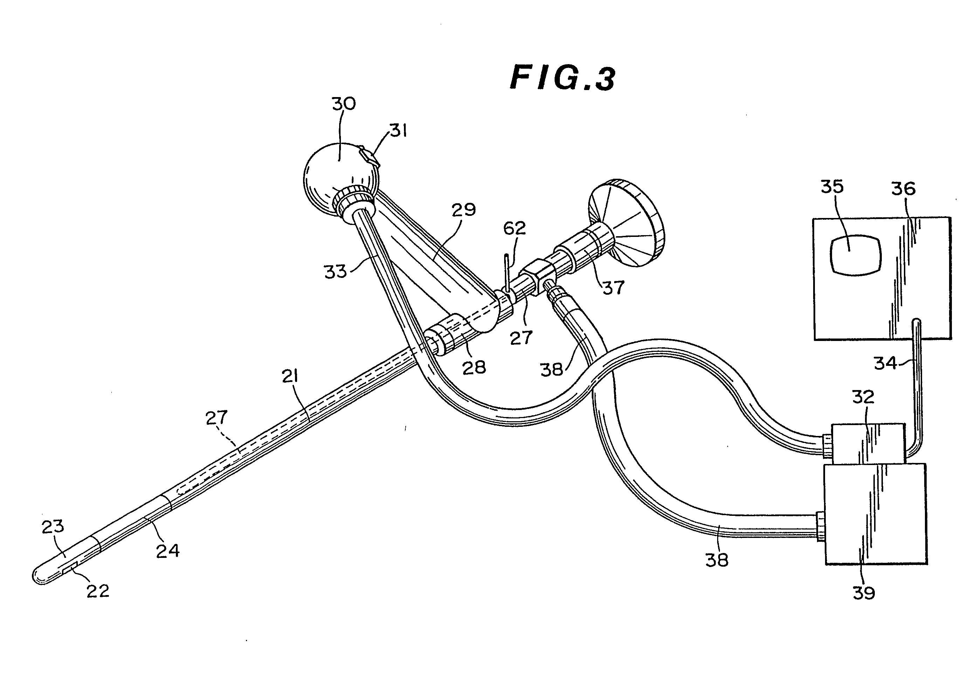

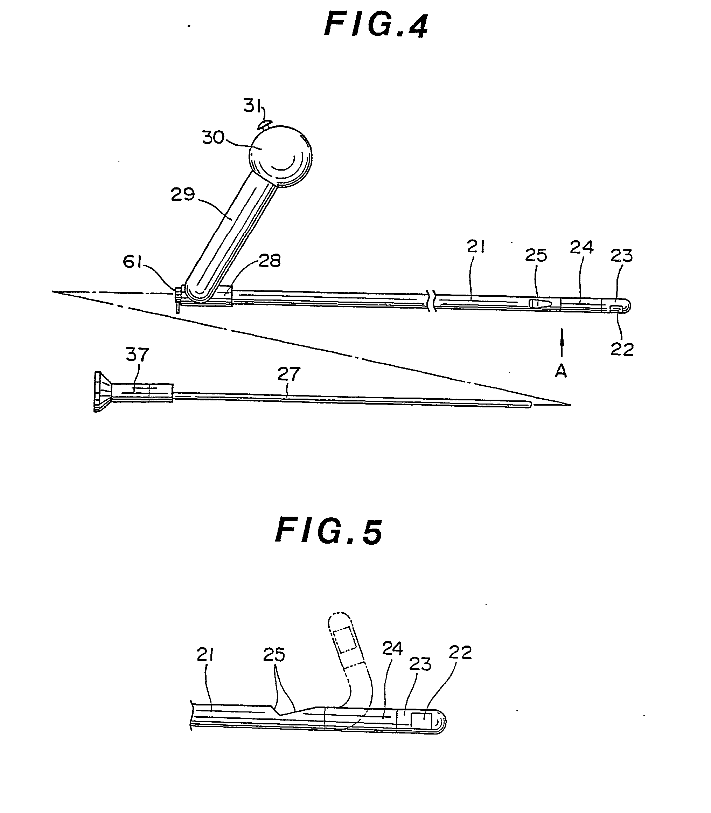

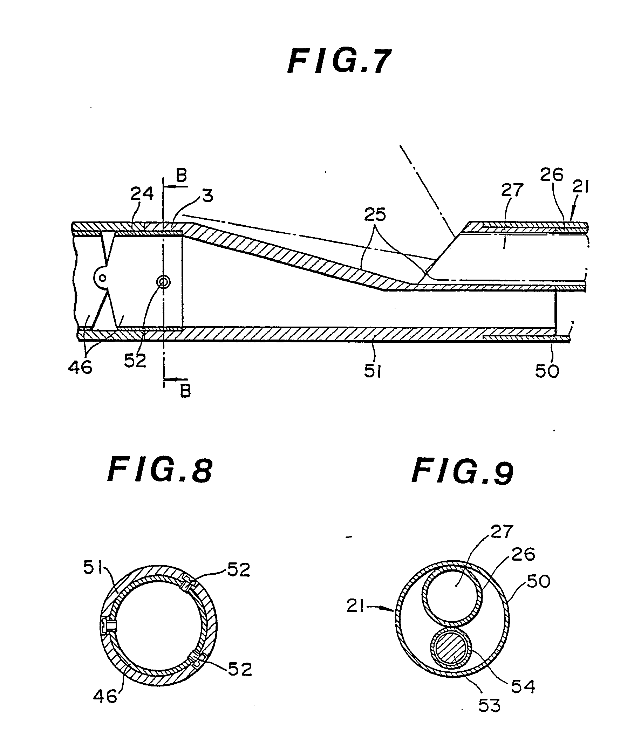

An exemplary embodiment of a device constructed in accordance with the present invention will now be described with reference to Figures 3 to 12. In these drawings, an insertion sheath 21 carries a probe tip 23 having a window 22 for transmitting and receiving ultrasonic waves on a side face, and a curving part 24 connects the front of the insertion sheath 21 to the probe tip 23, the sheath 21, probe tip 23 and curving part 24 all being substantially equal in diameter. An insert window 25 (Figure 4, Figure 7) is provided in the outer periphery of the insertion sheath 21 on the same side as the window 22, but to the rear of the curving part 24 to give a field of vision in a lateral direction, and an endoscope guide tube 26 is arranged to extend from this inset window 25 back to the rear end of the insertion sheath 21. A perspective endoscope 27 of a fine diameter is telescopically inserted into the guide tube 26 in this embodiment. The illustrated device is an abdominal cavity internal organ diagnostic device, and the inset window 25 is formed in the insertion sheath 21 so that the tip of the perspective endoscope 27 may be positioned in the field of view of this inset window 25 of the endoscope. The above-mentioned perspective endoscope 27 has a field of view with an included angle of at least 45°. The inset window 25 is formed in the sheath 21 to give the edge of the field of view an angle of inclination of 5° to 25° with respect to the longitudinal axis, so that the field of view of the endoscope 27 is not intercepted by the outer periphery of the insertion sheath 21.

A hand grip mount 28 is connected to the rear end of the insertion sheath 21. A grip 29 inclined forwardly at an angle of about 60° with respect to the longitudinal axis is integrally connected to the outer periphery of the upper part of this hand grip mount in the normal operating state, so that it can be gripped with one hand by an operator, and so that the grip 29 will not contact the body wall and will not obstruct the manipulation of the device, even if the insertion sheath 21 needs to be much inclined during insertion into a body cavity to diagnose a remote internal organ. The head of this grip 29 has a substantially spherical curve-control member 30 on which a curvature operating lever 31 is provided to project so as to be operable by the thumb when a hand is grasping the grip 29.

A driving means 32 containing a motor for producing a scanning motion of ultrasonic waves is formed separately from the grip 29, and is connected thereto via a flexible drive cable 33. Further, the driving means 32 is connected by a signal cable 34 to an indicating means 36 provided with an oscilloscope 35. A flexible light guide 38 is connected in front of an eyepiece 37 of the perspective endoscope 27 to feed in light from a light source 39. This light guide 38 and the drive cable 33 are connected in the same lateral direction with respect to the eyepiece 37 of the endoscope and the curve-control member 30 at the head of the grip 29, to improve operatability and avoid any interference during insertion of the sheath 21 into the body cavity, or the contacting operation placing the probe tip 23 against a predetermined internal organ for the diagnosis using ultrasonic waves.

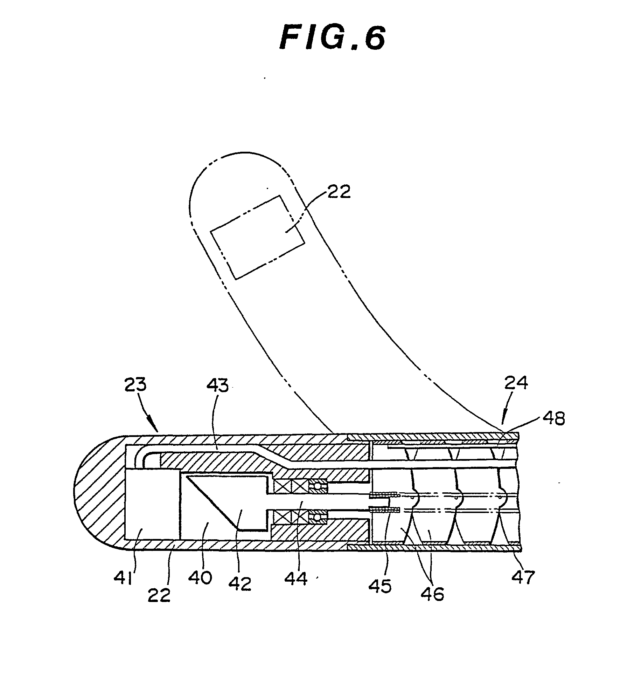

The probe tip 23 and curving part 24 are formed as shown in Figure 6, a medium chamber 40 is formed in the ultrasonic wave probe tip 23. An ultrasonic oscillator 41 and a mirror 42 are arranged opposed each other within a transmission medium chamber 40. A signal lead 43 extending through the insertion sheath 21 from the driving means 32 is connected to this oscillator 41. A shaft 44 is formed on the mirror 42. A flexible drive shaft 45 is connected to this shaft 44. The curving part 24 is formed by pivoting together a plurality of articulated members 46 which are each provided with a rubber coating 47. An operating wire 48 inserted through the curvature control member 30 is fixed to the foremost articulated member 46.

The bending direction of this curving part 24 is set to lie in the same lateral direction as the field of view of the endoscope 27, that is, to the right as seen by the operator in the normal operational state, so that the window 22 which serves for transmitting and receiving ultrasonic waves may be within the field of view of the endoscope 27, as shown in Figure 12. Thus, in the illustrated device for diagnosing internal organs within the abdominal cavity, the direction of the field of view of the endoscope 27 inserted in the insertion sheath 21 and the bending direction of the curving part 24 are set to the right as seen from the operator because, in the case of using the device to perform a diagnosis on an internal organ 49 within an abdominal cavity, that part of the skin containing many blood vessels and that position at which the internal organ is located are naturally known, and therefore the inserting position and inserting direction are medically predetermined, and with the field of view to the right in the normal operating state and with the curving part bent in the direction of the field of view, the device will be adapted to operate to permit observation of the internal organ and the close contact of the probe tip 23 with the internal organ. If the device is to be used to diagnose internal organs other than those within the abdominal cavity, then the direction of the field of view of the endoscope and of the curved direction need not be to the right, considered from the operator, provided always that the window 22 in the probe tip 23 is arranged at the same side as the endoscope window 25, and the direction of bending of the curving part is in the same direction.

As shown in Figure 7, the inset window 25 formed in the insertion sheath 21 is in a connecting member 51 connecting the tip of an outer tube 50 forming part of the insertion sheath 21 to the curving part 24. This connecting member 51 is fitted and fixed to the inner face of the outer tube 50, and at its end adjacent the curving part 24 is secured to the last articulated member 46 by screws 52 screwed in from the outer periphery as shown in Figure 8. This formation ensures that there is the widest possible space inside the connecting part of the connecting member 51 available to accommodate the drive shaft 53, signal cable 43 and operating wire 48, together with each articulated member 46. To accommodate the inset window 25 formed in the sheath 21, the mirror driving shaft 53 inserted through the outer tube 50 of the sheath is arranged on the side opposite the direction of the field of view of the endoscope 27, as shown in Figure 9.

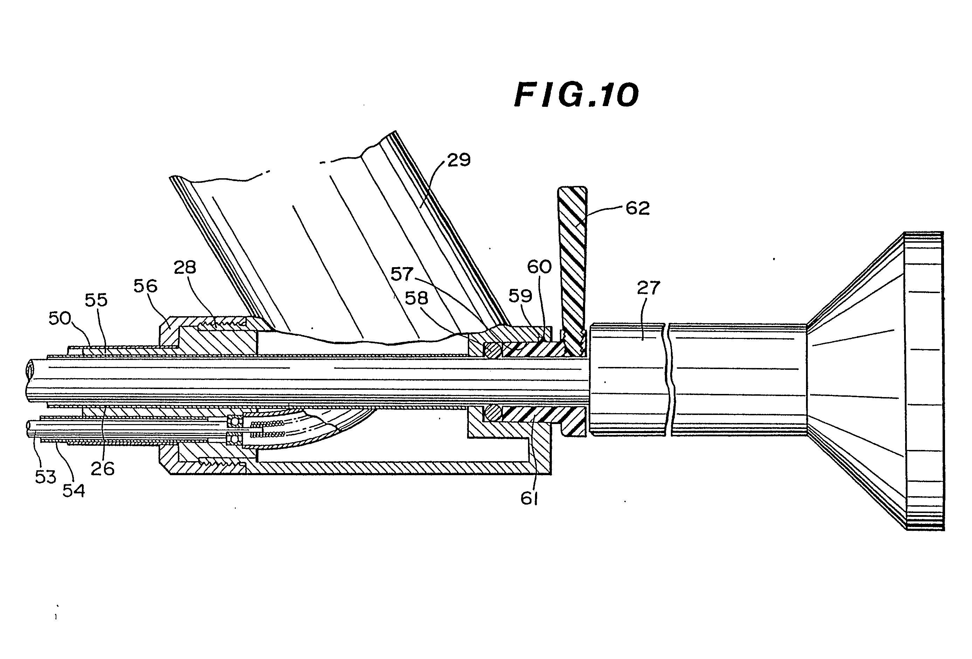

Further, the connection of the sheath 21 to hand grip mount 28 is as shown in Figure 10. A pipe receiver 55 holding and fixing the endoscope guide tube 26, drive shaft tube 54 and the sheath outer tube 50 is fitted within the hand grip mount 28, a male screw thread is formed on the outer periphery of this hand grip body 28 and a cap 56 having a female screw thread engages on the male screw thread to secure the pipe receiver 55 to the hand grip mount 28. A fixing hole 57 communicating with the endoscope guide tube 26 is formed at the rear of the hand grip mount 28. An O-ring 58 engaging with the outer periphery of the endoscope 27 is inserted in this fixing hole 57 and secured in place by an O-ring fastening body 61 having a projecting pin 60 engaged in a spiral groove 59 provided on the inner periphery within the hole 57, so that the body 61 can be screwed into the fixing hole 57 to press and deform the O-ring 58. The endoscope 27 is held in place by this deformed O-ring to prevent axial or rotating movement and form an airtight seal. The ring fastening body 61 is formed to receive the endoscope 27 and is provided with a lever 62 that projects outwardly at the rear.

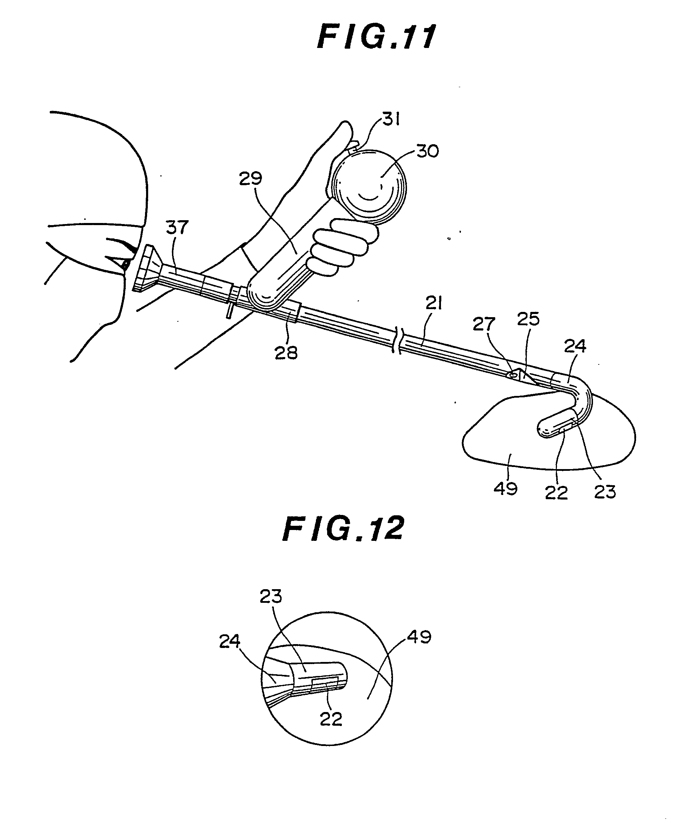

With such a formation, then as is shown in Figure 11, the grip 29 can be held with one hand by the operator and the sheath 21 inserted into a body cavity, for example, an abdominal cavity through a trocar (not illustrated) with the inside of the thumb applied to the curvature operating lever 31 of the substantially spherical curve-control member 30 formed at the head of the grip 29. In such case, the probe tip 23 is guided to an internal organ while, for example, the diagonal front in the right direction of the sheath 21 is being observed via the perspective endoscope 27 from the rear of the curving part 24. After the location of the internal organ 49 is confirmed, the curvature operating lever 31 is operated by thumb-pressure to pull the operating wire 48 and cause the curving part 24 to curve in the direction of the field of view of the endoscope 27, to the right as seen from the operator when dealing with the abdominal cavity interior, and thus the probe tip 23 and window 22 are brought into the field of view and the window 22 can be viewed as it is brought into close contact with a predetermined part of the internal organ 49, as shown in Figure 12.

As the endoscope 27 is made to fit up to the inset window 25 formed in the sheath 21 to the rear of the curving part 24 and has a perspective view to the right of the tip, the probe tip 23 and curving part 28 can be inserted without intercepting the field of view to the internal organ, and when the probe tip 23 is moved into the field of view of the endoscope 27 by the action of the curving part 24, the operation of close contacting the internal organ 49 can be effected while both the internal organ 49 and probe tip 23 are being viewed, so that the internal organ can be accurately and easily examined. Also, the position of contact of the window 22 for transmitting and receiving ultrasonic waves with relation to the internal organ can be confirmed.

In the described embodiment the endoscope 27 is made as a separate unit that can be telescopically inserted into the insertion sheath 21, so that other available fine diameter endoscopes can be used, and an endoscope can be repaired or replaced in the event of failure. In alternative embodiments the endoscope may be integrally contained in the insertion sheath, to avoid the need to separately sterilise the endoscope tube or parts.

Our co-pending European Patent Application No. 82 301 452.7 (Specification No. 0 061 331) of even date claims some of the subject matter disclosed herein.

## Claims

1. A combined endoscope and ultrasonic diagnostic device wherein the endoscope is incorporated in a housing carrying an ultrasonic probe, which housing comprises an elongate insertion sheath (21) connected by a bendable curving part (24) to a distal probe tip (23) which has a first, peripheral window (22) for transmitting and receiving ultrasonic waves when in contact with an internal organ within a body cavity, the insertion sheath, probe tip and curving part all being of substantially equal diameter, characterised in that a second, peripheral window (25) is incised in the outer periphery of the insertion sheath proximally of the above-mentioned curving part in a position to give a view of the probe tip and first window, when said curving part is bent, and that the endoscope is housed within the insertion sheath to extend from said second window to the proximal end of the sheath, whereby bending of the curving part may be effected to make the first window (22) visible through the second window (25).

2. A device as claimed in Claim 1, in which said endoscope is constructed as an integral structure with said insertion sheath.

3. A device as claimed in Claim 1, in which said endoscope is constructed as a unit which is telescopically insertable into said insertion sheath.

4. A device as claimed in any preceding Claim, characterised in that the direction of the field of view of the endoscope and of the ultrasonic wave transmitting and receiving window provided in the probe tip are arranged in directions mutually intersecting at right angles.

5. A device as claimed in any preceding Claim, characterised in that the direction of the field of view of the endoscope and the bending direction of the curving part are set to the right, as seen from the operators position.

## Revendications

1. Dispositif combiné d'endoscopie et de diagnostic à ultrasons, dans lequel l'endoscope est incorporé dans un boîtier portant une sonde à ultrasons, ledit boîtier comportant un manchon d'insertion allongé (21) relié par une partie recourbable (24) à un embout d'extrémité de sonde (23) qui possède une première fenêtre périphérique pour transmettre et recevoir des ondes ultrasonores lorsqu'elle est en contact avec un organe interne dans une cavité du corps, le manchon d'insertion, l'embout de sonde et la partie recourbable possédant sensiblement le même diamètre, caractérisé par le fait qu'une seconde fenêtre périphérique (25) est découpée dans la périphérie externe du manchon d'insertion à proximité de ladite partie recourbable dans une position permettant de donner une vue de l'embout de sonde et de la première fenêtre lorsque ladite partie recourbable est recourbée et que l'endoscope est logé dans le manchon d'insertion de manière à s'étendre depuis ladite seconde fenêtre jusqu'à l'extrémité arrière du manchon, la partie recourbable pouvant ainsi être recourbée de manière à rendre la première fenêtre (22) visible à travers la seconde fenêtre (25).

2. Dispositif selon la revendication 1, dans lequel ledit endoscope est réalisé sous forme d'une structure d'un seul tenant avec le manchon d'insertion.

3. Dispositif selon la revendication 1, dans lequel ledit endoscope est réalisé sous la forme d'un ensemble susceptible d'être inséré de manière télescopique dans ledit manchon d'insertion.

4. Dispositif selon l'une quelconque des revendications précédentes, caractérisé par le fait que la direction du champ de vision de l'endoscope et de la fenêtre de transmission et de réception d'ondes ultrasonores réalisée dans l'embout de sonde sont disposées dans des directions se coupant mutuellement à angle droit.

5. Dispositif selon l'une quelconque des revendications précédentes, caractérisé par le fait que la direction du champ de vision de l'endoscope et la direction de courbure de la partie recourbable sont orientées vers la droite en se plaçant dans la position des opérateurs.

## Patentansprüche

1. Eine Verbindung eines Endoskops mit einem Ultraschalldiagnostikgerät, worin das Endoskop in einem Gehäuse eingeschlossen ist, das eine Ultraschall-Sonde trägt, welches Gehäuse einen langgestreckten Einsatzmantel (21) einschliesst, der durch einen biegsamen Kurventeil (24) mit einer distalen Sondenspitze (23) verbunden ist, die ein erstes, peripherisches Fenster (22) hat zum Aussenden und Empfangen von Ultraschallwellen, wenn ein Kontakt mit einem inneren Organ in eine Körperaushöhlung erfolgt, wobei der Einsatzmantel, die Sondenspitze und der Kurventeil alle einen im wesentlichen gleichen Durchmesser haben, dadurch gekennzeichnet, dass ein zweites peripherisches Fenster (25) in dem äusseren Umfang des Einsatzmantels proximal von dem oben genannten Kurventeil in einer solchen Lage eingeschnitten ist, dass es einen Blick auf die Sondenspitze und das erste Fenster gewährt, wenn der genannte Kurventeil gebogen wird, und dass das Endoskop innerhalb des Einsatzmantels untergebracht ist, um sich von dem genannten zweiten Fenster zu dem proximalen Ende des Mantels zu erstrecken, wodurch ein Biegen des Kurventeils bewirkt werden kann, um das erste Fenster (22) durch das zweite Fenster (25) sichtbar zu machen.

2. Eine Geräteverbindung gemäss Anspruch 1, in der das genannte Endoskop als eine integrale Einheit mit dem Einsatzmantel gestaltet ist.

3. Eine Geräteverbindung gemäss Anspruch 1, in der das genannte Endoskop als eine Einheit gestaltet ist, die teleskopisch in den genannten Einsatzmantel einsetzbar ist.

4. Eine Geräteverbindung gemäss einem jeden der vorgenannten Ansprüche, dadurch gekennzeichnet, dass die Richtungen der Blickfelder des Endoskops und des Ultraschall Sende- und Empfangsfensters in der Sondenspitze sich gegenseitig rechtwinklig kreuzend angeordnet sind.

5. Eine Geräteverbindung gemäss einem jeden der vorgenannten Ansprüche, dadurch gekennzeichnet, dass die Richtung des Blickfeldes des Endoskops und die Biegerichtung des Kurventeils nach rechts, vom Benutzer aus gesehen, gesetzt sind.

# FIG.1

# FIG.2

# FIG.3

0 061 332

# FIG.4

# FIG.5

# FIG.6

# FIG.7

# FIG.8

# FIG.9

# FIG.10

# FIG.11

# FIG.12